# EUROPEAN PATENT SPECIFICATION

(11) **EP 2 144 552 B1**
(45) Date of publication and mention of the grant of the patent: **13.03.2013**
(21) Application number: 08754032.4
(22) Date of filing: 05.05.2008
(51) Int. Cl.: A61B 3/135

(54) **WHITE LED MODULE FOR EASY REPLACEMENT OF BULB MODULE OF SLIT LAMP**
WEISSES LED-MODUL FÜR EINFACHES AUSWECHSELN EINES GLÜHLAMPENMODULS EINER SPALTLAMPE
MODULE DE DEL BLANCHE POUR REMPLACEMENT AISE DU MODULE D'AMPOULE D'UNE LAMPE A FENTE

(30) Priority: 05.05.2007 SI 200700105
(43) Date of publication of application: 20.01.2010
(73) Proprietor: Optotek d.o.o., 1000 Ljubljana (SI)
(72) Inventor: VEDLIN, Boris, 1000 Ljubljana (SI); ZALAR, Matjaz, 1294 Grosuplje (SI)
(74) Representative: Flak, Antonija
(86) International application number: PCT/SI2008/000026
(87) International publication number: WO 2008/136779

(56) References cited:
- EP-A- 1 602 323
- US-A1- 2004 183 482
- US-A1- 2006 012 997
- US-A1- 2006 291 256

## Description

### Field of the Invention

The invention belongs to the field of medical devices used in ophthalmology, more precisely to the field of illumination of eyes during eye examinations by means of a slit lamp.

### Technical Problem

The technical problem is a construction solution with a strong white LED module that will replace a bulb module in the existing diagnostic slit lamps manufactured by Haag-Streit, whereby an equal illumination over the entire aperture, i.e. slit, should be provided. The construction solution must allow a replacement of a light source by simultaneously preserving mechanical, optical and electric compatibility between the white LED and the bulb with unchanged functions of a slit lamp. The light source should be easily replaceable allowing a physician to replace it before or during eye examination. It must also allow a replacement of the light source in other slit lamps of similar types, in which the light source is provided above the lens of the slit lamp. Since the set technical problem concerns the modernisation of the existing slit lamps that usually have an incremental current regulation, the optimum positioning of the regulator should be solved in a way to allow for a linear regulation of the supply current of the light source.

### Prior Art

Slit lamps are devices used in eye examinations. A physician can visually examine individual eye parts by means of an illumination slit created by such lamp. In a majority of known types of slit lamps the slit is illuminated by a bulb, most often a halogen lamp. Due to high power consumption such lamps tend to heat, and what's more they have a limited transmission. They are very sensitive and their heating contributes to a short life, which can be even below 100 hours. Luminous intensity is set by the current flowing through the heating filament. In this way the temperature of the heating filament changes thus causing the light colour to change as well, which is very disturbing for the users of the lamp. In a majority of eye examinations a light bluish light is desired, said light being produced by a heating filament at high temperatures, i.e. at high luminous intensity, which significantly contributes to a shorter life of the bulb. From this point of view, a more suitable solution is that with a light source in the form of a white LED. With a slit lamp, in which the light source is a white LED, eye examination is improved and facilitated, since the illumination slit on the eye is white and equally illuminated and the colour of the light beam is not subject to changes due to luminous intensity. A further advantage is also a longer interval between maintenance interventions, because the life of a LED is much longer than that of a halogen bulb. By using LEDs in slit lamps the interval between maintenance interventions can be considerably extended, even up to 1000-times. We should also mention a considerably lower power consumption in comparison with halogen light sources. Lower power consumption means lower heating, which is generally undesirable. In known solutions to slit lamps manufactured by Haag-Streit and similar variants with a light source positioned above the lens of the slit lamp the bulb can only be replaced by a LED only by performing extensive mechanical, optical and electric modifications of the existing slit lamps and cannot be carried out by a physician or a serviceman.

US 2004/0183482 A1 discloses an adapter for use with a medical diagnostic instrument typically configured with an incandescent lamp as an illumination source and a power supply for powering said incandescent lamp, said adapter having means for permitting at least one white LED to be used as the illumination source for said instrument while permitting said existing power supply to used therewith.

The Slovene patent No. 21782 discloses a solution with a white LED, yet it is adapted to a new solution to the slit lamp and is not applicable for old slit lamps that need to be modernised.

### Solution to the Technical Problem

The essence of the white LED module as a light source for slit lamps of the invention lies in that a strong white LED is built into a special housing with cooling ribs that is to be inserted into an existing slit lamp of type Haag-Streit 3 instead of the bulb. The shape of the module provides mechanical, optical and electric compatibility with the existing slit lamp, whereby the electronic comprised within the module ensures proper operation of the white LED. The white LED is supplied via existing regulation power supply, usually with discrete current settings used to supply power to the bulb. Fine regulation of electric power on the white LED is achieved by a specially shaped electronic block positioned between the power supply and the white LED, said block being connected to the position of the supply cable.

The bulb module of the invention can be replaced by a physician by removing the cover on the light tower of the slit lamp, removing the bulb, inserting a module to the position of the bulb and re-placing the cover. The physician or the person changing the light source needs no additional tools.

The white LED module as a light source for slit lamps of the invention is described in more detail in the continuation by way of the following figures:

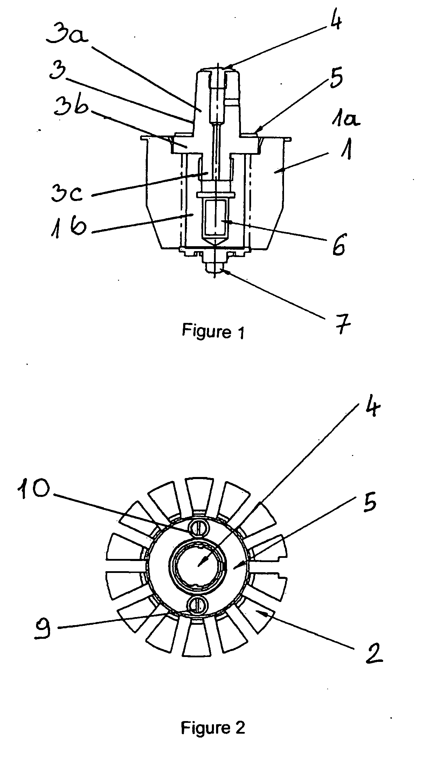

- Figure 1 -: a white LED module
- Figure 2 -: plan view of the white LED module

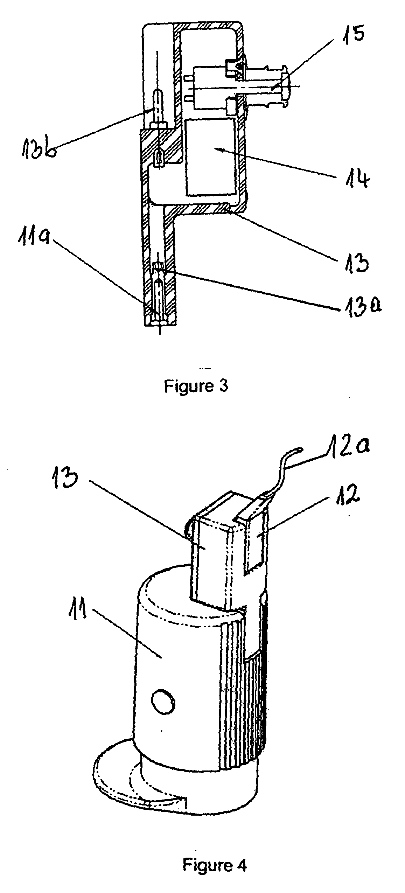

- Figure 3 -: electronic block for fine regulation of supply current
- Figure 4 -: cover of the module of the invention together with the electronic block in a housing 13
- Figure 5 -: schematic view of the existing slit lamp with the integrated module of the invention and the electronic block in the housing 13

A white LED module as a light source for slit lamps of the invention is characterised in that its height and width equal those of the bulb module in old slit lamps manufactured by Haag-Streit, whereby electric contact supply positions are provided on the same positions as those in the existing lamps. It is herewith ensured that the cover remains unchanged. A supply cable or an electronic block plug for fine regulation of the white LED illumination can be inserted into the cover socket, said block being connected to the existing regulation power supply of the slit lamp via electronic circuit.

The white LED module consists of a housing 1 with ribs 2 for cooling, of a cylindrical casing 3 with a contact site 4 on the top of part 3a of the casing 3 and a contact site 5 on an extended part 3b of the casing 3. A housing of an electronic assembly 6 is arranged on the third part 3c of the casing 3, said housing allowing for proper operation of a white LED 7 within standard voltages received from a regulation power supply 24. The interior of the housing 1 is shaped as a wide cylinder 1a in its upper part, whereas its lower part 1b has a shape of a narrow cylinder, whose height is approximately 5/6 of the height of the housing 1. The casing 3 is screwed to the housing 1 with screws 9 and 10. A holder 8 of the white LED 7 is fastened to the bottom surface of the housing 1. The ribs 2 of the housing are equidistantly arranged on the circumference of the housing 1. The number of the ribs 2 totals 14, yet the number may vary from 4 to 20. The housing 1 with the ribs 2 is positioned in the interior of the slit lamp in a way that the external edges 2a of the ribs 2 fit onto the holder of the bulb module after the latter has been removed. The rib 2 is shaped in a way that the upper surface, which terminates with the edge 2a, is trapezoidal. The rib 2 somewhat narrows along the edge 2a and is parallel to the axis at approximately three fifths of the rib height, where it narrows diagonally to the lower diameter of the housing 1.

The white LED module of the invention is covered by a cover 11 having in its upper part a socket to connect a supply cable 12a of the plug 12. Should fine illumination regulation of the LED 7 be required, cylindrical recesses of a housing 13 are attached to the pins 11a of the socket of the cover 11, said recesses supplying power via pins 13a and via contact with pins 11 a to the white LED 7. The upper part of the housing 13 is shaped in the form of a hollow rectangle, into which an electronic block 14 and an illumination regulation knob 15 are arranged. On the axis of the pins 11 a and the pins 13a there are also pins 13b to connect the plug 12. The shape of the housing with pins for electrical connection of the power supply 24, of the electronic block 14 in the housing 13 and of the socket 12 allows for a simple addition of a white LED illumination regulator. The regulating electronic circuit may be provided in a variety of ways and is not subject of the present invention.

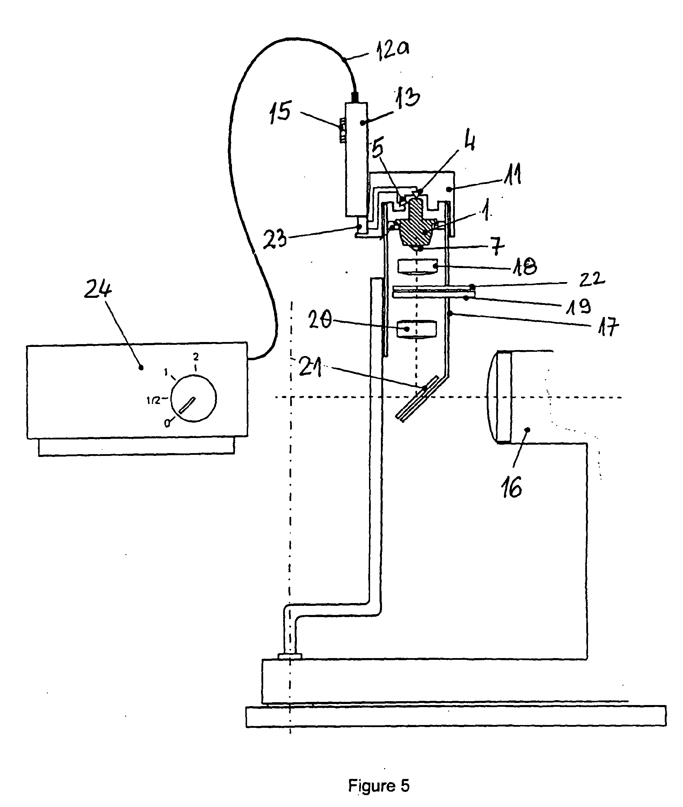

The basic shape of the slit lamp 16 manufactured by Haag-Streit or of a similar variant with a light source lying above the lens of the slit lamp as shown in Figure 5 has a light source in the form of a bulb arranged in a light tower 17. After the bulb is removed, the module of the invention is arranged in this place. All other components of the slit lamp 16 remain unchanged, i.e. the same as prior to the exchange of the bulb module of the invention. The light from the white LED 7 flows as bulb light via capacitor optics 18 through a plate 19 with a slit, which reflects same via output lens 20 and the mirror to a patient's eye.

The white LED 7 may be the so-called cold white LED having predominance in the blue part of the radiation spectrum and is convenient when watching details in an eye. The so-called warm white LED may be used as a light source having a more equal distribution in the radiation spectrum and its light is similar to that obtained from a halogen bulb. The colour of the LED does not change with luminous intensity in any of both solutions.

Owing to differences in the light colour between the cold white LED and a conventional halogen bulb it is recommendable that the first replacement of the bulb module of the invention comprising such white LED in the slit lamp is performed by a serviceman, yet this is not necessary due to the intervention being very simple.

When the bulb is replaced by module 2 comprising the strong white LED 7, an insertion of a special "red-free" filter into a rotatable filter plate 22 in front of the plate 19 with a slit is foreseen. The rotatable plate 22 already has a special spot for an additional, special filter. This special red-free filter is adapted to the wavelength of the white LED and causes the image of the slit from the plate 19 to reflect to the examined eye only in the green part of the light spectrum, which is desirable especially when examining eye's retina.

The module of the invention is designed in a way to fit into an existing bulb bearing 23. Moreover, the shape of the module of the invention allows for the electric pins 11 a on the cover 11 needed to supply power to the bulb to supply power to the white LED 7 after the exchange of the light source. Cooling of the module of the invention, i.e. of the white LED 7, is provided via the same cooling system as used for bulb cooling and has the same radiation angle. The mechanical compatibility of the module of the invention with the existing slit lamp 16 is thus provided for. Optical compatibility between the module of the invention and the existing slit lamp 16 is achieved by the spot of building in the white LED 7 in the module of the invention. The white LED 7 is integrated in a way that after the module of the invention is arranged in the slit lamp 16, the position of the white LED 7 in the height of the light tower 17 equals that of the original position of the bulb's filament. With the module of the invention and the cover 11 being positioned, the white LED 7 received power from the same regulation power supply 24 with discrete power settings as used to supply power to the bulb. The pin 12 on the cover 11 and the supply cable 12a remain unchanged. Thus, electrical compatibility of the module of the invention with the existing slit lamp 16 is achieved.

A conventional regulation power supply 24 used to supply power to the bulb usually has discrete settings, e.g. four possible discrete power settings: off, half power/current operation, nominal power/current operation and increased power/current operation. Fine regulation of white LED illumination can be achieved by an upgrading of the module of the invention by the electronic block 14. Electric current coming from the regulation power supply 24 is further finely regulated by means of the electronic block 14, which can be positioned between the regulation power supply 24 and the strong white LED 7 on the cover 11. The housing 13 with its electronic regulator 14 is thus mechanically compatible with the slit lamp 16. It means it can be mechanically inserted between the pin 11 a on the cover 11 and the supply cable 12 from the regulation power supply 24 manually without using any other tool. Current intensity between the value 0 and the highest value set on the regulation control 15 can be linearly set on the electronic block in the housing 13 by means of the rotating knob 15. The housing 13 with the electronic regulator 14 can also be used in the mode described above, when the slit lamp 16 is devoid of the regulation power supply 24 and only possesses a supply transformer.

## Claims

1. Module with a white LED as a light source for slit lamps that replaces a bulb as a light source in slit lamps manufactured by Haag-Streit, wherein the module consists of a housing (1) with ribs (2) for cooling, of a cylindrical casing (3) with a contact site (4) on the top of part (3a) of the casing (3) and a contact site (5) on an extended part (3b) of the casing (3), wherein a housing of an electronic assembly (6) is arranged on the third part (3c) of the casing (3), said housing allowing for proper operation of a white LED (7) within standard voltages received from a regulation power supply (24), wherein the interior of the housing (1) is shaped as a wide cylinder (1 a) in its upper part, whereas its lower part (1 b) has a shape of a narrow cylinder, whose height is approximately 5/6 of the height of the housing (1), wherein the casing (3) is screwed to the housing (1) with screws (9 and 10), wherein a holder (8) of the white LED (7) is fastened to the bottom surface of the housing (1), wherein the ribs (2) of the housing are equidistantly arranged on the circumference of the housing (1), wherein the number of the ribs (2) totals 14, wherein the housing (1) with the ribs (2) is positioned in the interior of the existing slit lamp in a way that the external edges (2a) of the ribs (2) fit onto the holder of the bulb module after the latter has been removed, wherein the rib (2) is shaped in a way that the upper surface, which terminates with the edge (2a), is trapezoidal, in that the rib (2) somewhat narrows along the edge (2a) and is parallel to the axis at approximately three fifths of the rib height, where it narrows diagonally to the lower diameter of the housing (1).

2. Module with a white LED as a light source for slit lamps as claimed in Claim 1, **characterised in that** it can be upgraded by an electronic block (14) in the housing (13) allowing for a fine linear illumination regulation of the white LED (7) apart from the existing regulation power supply (24), **in that** a cover (11) having in its upper part a socket to connect a supply cable (12a) of the plug (12), **in that** cylindrical recesses of a housing (13) are attached to the pins (11a) of the socket of the cover (11), said recesses supplying power via pins (13a) and via contact with pins (11 a) to the white LED (7), **in that** the upper part of the housing (13) is shaped in the form of a hollow rectangle, into which the electronic block (14) and an illumination regulation knob (15) are arranged, **in that** on the axis of the pins (11 a) and the pins (13a) there are also pins (13b) to connect the plug (12), **in that** the shape of the housing (13) with pins for electrical connection of the power supply (24), of the electronic block (14) and of the socket (12) allows for a simple addition of a white LED (7) illumination regulator.

3. Module with a white LED as a light source for slit lamps as claimed in Claim 1 and 2, **characterised in that** it can also be used in other variants of slit lamps, in which a light source lies above the lens of the slit lamp.

4. Module with a white LED as a light source for slit lamps as claimed in Claim 3, **characterised in that** it provides for a use of the electronic block (14) in the housing (13) for fine illumination regulation of the white LED (7) also in case when the slit lamp (16) only has a supply transformer.

5. Module with a white LED as a light source for slit lamps as claimed in any of Claims 1 to 4, **characterised in that** a cold or hot white LED is used as the white LED (7).

6. Module with a white LED as a light source for slit lamps as claimed in Claim 1, **characterised in that** the number of ribs (2) can vary from 2 to 20.

## Patentansprüche

1. Modul mit einer weißen LED als Lichtquelle für Spaltlampen, das eine Glühlampe als Lichtquelle in von Haag-Streit hergestellten Spaltlampen auswechselt, umfassend ein Gehäuse (1) mit Rippen (2) für Kühlung, eine zylindrische Hülse (3) mit einer Kontaktstelle (4) oberhalb des Teils (3a) der Hülse (3) sowie eine Kontaktstelle (5) an einem erstreckten Teil (3b) der Hülse (3), wobei das Gehäuse einer elektronischen Anordnung (6) am dritten Teil (3c) der Hülse (3) angeordnet ist und das Gehäuse einen richtigen Betrieb einer weißen LED (7) innerhalb von den aus einer Regulierungs-Energieversorgung (24) empfangenen Standard-Spannungen ermöglicht, wobei das Innere des Gehäuses (1) in seinem Oberteil als ein breiter Zylinder (1 a) ausgebildet ist und sein Unterteil (1 b) die Form eines engen Zylinders, dessen Höhe etwa 5/6 der Höhe des Gehäuses (1) erreicht, aufweist,wobei die Hülse (3) mit Schrauben (9 und 10) in das Gehäuse (1) eingeschraubt ist, wobei ein Halter (8) der weißen LED (7) an der Bodenfläche des Gehäuses (1) befestigt ist, wobei die Rippen (2) des Gehäuses am Umfang des Gehäuses (1) in gleichen Abständen angeordnet sind, wobei die Gesamtzahl der Rippen (2) 14 beträgt, wobei das Gehäuse (1) mit den Rippen (2) im Inneren der vorhandenen Spaltlampe so angeordnet ist, dass die Außenkanten (2a) der Rippen (2) an den Halter des Glühlampenmoduls, nachdem der letztere entfernt worden ist, anliegen, wobei die Rippe (2) so ausgebildet ist, dass die mit der Kante (2a) abgeschlossene Oberfläche trapezoidförmig ist, und die Rippe (2) sich ein wenig entlang der Kante (2a) verjüngt und parallel mit der Achse bei etwa drei Fünftel der Rippenhöhe kommt, wo sie sich schräg zum unteren Durchmesser des Gehäuses (1) verjüngt.

2. Modul mit einer weißen LED als Lichtquelle für Spaltlampen nach Anspruch 1, **dadurch gekennzeichnet, dass** es mit einem elektronischen Block (14) in einem Gehäuse (13) aufrüstbar ist, der eine genaue kontinuierliche Beleuchtungsstärke-Regulation der weißen LED (7) neben der vorhandenen Regulations-Energieversorgung (24) ermöglicht, dass ein Deckel (11) an seinem Oberteil eine Steckdose zur Zuschaltung eines Versorgungskabels (12a) des Steckers (12) aufweist, dass zylindrische Ausnehmungen des Gehäuses (13) auf Anschlussstifte (11a) der Steckdose des Deckels (11) aufgesteckt sind, wobei die Ausnehmungen die weiße LED (7) mit Energie durch Anschlussstifte (13a) und durch ein Kontakt mit Anschlussstiften (11a) versorgen, dass das Oberteil des Gehäuses (13) in Form eines hohlen Rechtecks, in dem der elektronische Block (14) und ein Knopf (15) zur Beleuchtungsstärke-Regulation angebracht sind, ausgebildet ist, dass an der Achse der Anschlussstifte (11a) und der Anschlussstifte (13a) auch die Anschlussstifte (13b) zur Verbindung des Steckers (12) angebracht sind, dass die Form des Gehäuses (13) mit Anschlussstiften zur elektrischen Verbindung der Energieversorgung (24), des elektronischen Blocks (14) und der Steckdose (12) ein einfaches Zufügen des Beleuchtungsstärke-Regulators der weißen LED (7) ermöglicht.

3. Modul mit einer weißen LED als Lichtquelle für Spaltlampen nach Anspruch 1 und 2, **dadurch gekennzeichnet, dass** es auch in anderen Varianten von Spaltlampen, in denen die Lichtquelle oberhalb des Objektivs der Spaltlampe liegt, Anwendung finden kann.

4. Modul mit einer weißen LED als Lichtquelle für Spaltlampen nach Anspruch 3, **dadurch gekennzeichnet, dass** es Anwendung eines elektronischen Blocks (14) im Gehäuse (13) für eine genaue Beleuchtungsstärke-Regulation der weißen LED (7) auch dann ermöglicht, wenn die Spaltlampe (16) nur einen Versorgungsumwandler aufweist.

5. Modul mit einer weißen LED als Lichtquelle für Spaltlampen nach irgendwelchem der Ansprüche 1-4, **dadurch gekennzeichnet, dass** es sich bei der weißen LED (7) um eine kalte oder heiße LED handelt.

6. Modul mit einer weißen LED als Lichtquelle für Spaltlampen nach Anspruch 1, **dadurch gekennzeichnet, dass** die Zahl von Rippen (2) von 2 bis 20 betragen kann.

## Revendications

1. Module avec une DEL blanche comme une source lumineuse pour lampes à fente qui remplace une ampoule comme une source lumineuse dans lampes à fente produites par Haag-Streit consistant en un boîtier (1) avec nervures (2) de refroidissement, une douille (3) cylindrique avec un point de contact (4) au-dessus de la partie (3a) de la douille (3) et un point de contact (5) sur une partie étendue (3b) de la douille (3), le boîtier d'un agencement électronique (6) étant disposé sur la troisième partie (3c) de la douille (3), ladite douille permettant une opération propre de la DEL (7) blanche à l'intérieur de tensions standards reçues de l'alimentation (24) de régulation, l'intérieur du boîtier (1) étant formé comme un cylindre large (1 a) à sa partie supérieure, tandis que sa partie inférieure (1 b) a la forme d'un cylindre étroit, dont la hauteur s'élève à environ 5/6 de la hauteur du boîtier (1), la douille (3) étant fixée sur le boîtier (1) avec vis (9 et 10), un cadre de support (8) de la DEL (7) blanche étant fixé sur la surface de fond du boîtier (1), les nervures (2) du boîtier étant disposées d'une manière équidistante sur la circonférence du boîtier (1), le nombre total de nervures (2) s'élévant à 14, le boîtier (1)avec les nervures (2) étant disposé à l'intérieur d'une lampe à fente existante en sorte que les bords extérieurs (2a) des nervures (2) vont sur le cadre de support (8) du module d'ampoule après que le dernier a été enlevé, la nervure (2) étant formée en sorte que la surface supérieure qui se termine avec le bord (2a) est d'une forme trapézoïdale, que la nervure (2) se rétrécit le long du bord (2a) et est en parallèle avec l'axe à environs trois cinquièmes de la hauteur de la nervure, où elle se rétrécit diagonalement vers le diamètre inférieur du boîtier (1).

2. Module avec une DEL blanche comme une source lumineuse pour lampes à fente selon la revendication 1, **caractérisé en ce qu'**il peut être modernisé avec un bloc électronique (14) dans le boîtier (13) permettant une régulation d'illumination linéaire juste de la DEL (7) blanche outre l'alimentation (24) de la régulation existante, **en ce qu'**il a un couvercle (11) doté sur sa partie supérieure d'une prise femelle pour la connection d'un cable d'alimentation (12a) d'une prise mâle (12), **en ce que** cavités cylindriques du boîtier (13) sont attachées aux broches (11a) de la prise femelle du couvercle (11), ledites cavités alimentant l'électricité via broches (1 3a) et via contacts avec broches (11a) à la DEL (7) blanche, **en ce que** la partie supérieure du boîtier (13) est formée comme un rectangle creux, dans laquelle sont disposés le bloc électronique (14) et un bouton pour la régulation d'illumination (15), **en ce que** sur l'axe des broches (11a) et des broches (13a) il se trouvent aussi broches (13a) pour la connection de la prise mâle (12), **en ce que** la forme du boîtier (13) avec les broches pour la connection électrique de l'alimentation (24), du bloc électronique (14) et de la prise femelle (12) permet une addition simple d'un régulateur d'illumination de la DEL (7) blanche.

3. Module avec une DEL blanche comme une source lumineuse pour lampes à fente selon la revendication 1 et 2, **caractérisé en ce qu'**il peut aussi être employé dans d'autres variantes des lampes à fente, dans lequelles la source lumineuse se trouve au-dessus de l'objectif de la lampe à fente.

4. Module avec une DEL blanche comme une source lumineuse pour lampes à fente selon la revendication 3, **caractérisé en ce qu'**il permet l'emploi du bloc électronique (14) dans le boîtier (13) pour la régulation d'illumination juste de la DEL (7) blanche même en cas où la lampe à fente (16) ne dispose que d'un transformateur d'alimentation.

5. Module avec une DEL blanche comme une source lumineuse pour lampes à fente selon quelconque de revendications 1-4, **caractérisé en ce qu'**une DEL froide ou chaude peut être employée comme la DEL (7) blanche.

6. Module avec une DEL blanche comme une source lumineuse pour lampes à fente selon la revendication 1, **caractérisé en ce que** le nombre de nervures (2) peut varier de 2 à 20.
